# NEUE EUROPÄISCHE PATENTSCHRIFT

(11) **EP 3 056 163 B2**
(45) Veröffentlichungstag und Bekanntmachung des Hinweises auf die Entscheidung über den Einspruch: **08.07.2020**
(45) Hinweis auf die Patenterteilung: 19.07.2017
(21) Anmeldenummer: 16153458.1
(22) Anmeldetag: 29.01.2016
(51) Int. Cl.: A61B 90/20, G02B 21/00, A61B 90/00, A61B 1/00, A61B 1/04, A61B 18/00, G02B 21/16, G02B 21/22, G02B 21/36

(54) **VISUALISIERUNGSSYTEM**
VISUALIZATION SYSTEM
SYSTEME DE VISUALISATION

(30) Priorität: 12.02.2015 DE 102015202605
(43) Veröffentlichungstag der Anmeldung: 17.08.2016
(73) Patentinhaber: Carl Zeiss Meditec AG, 07745 Jena (DE)
(72) Erfinder: STEFFEN, Dr. Joachim, 73463 Westhausen (DE); PANITZ, Gerald, 73479 Ellwangen (DE); HAUGER, Dr. Christoph, 73431 Aalen (DE); GUCKLER, Dr. Roland, 89081 Ulm (DE); FILIPPATOS, Konstantinos, 80802 München (DE)
(74) Vertreter: Gauss, Nikolai

(56) Entgegenhaltungen:
- JP-A- 2004 065 317
- JP-A- 2011 167 344
- US-A1- 2001 055 062
- US-A1- 2002 151 784
- US-A1- 2006 108 509
- US-A1- 2010 321 772

## Beschreibung

Die Erfindung betrifft ein Visualisierungssystem mit einem Operationsmikrogemäß des Anspruchs 1. Aus der JP 2004 065317 A ist ein Visualisierungssystem der eingangs genannten Art bekannt. In diesem Visualisierungssystem gibt es in Operationsmikroskop und ein Endoskop, das in einem ersten Betriebszustand in das Operationsmikroskop eingeführt werden kann und das in einem von dem ersten Betriebszustand verschiedenen weiteren Betriebszustand das Beobachten des Operationsbereichs neben dem Operationsmikroskop ermöglicht. Je nachdem ob das Endoskop innerhalb oder außerhalb des Operationsmikroskops betrieben wird, nimmt das Operationsmikroskop unterschiedliche Betriebszustände an.

Die US 2002/0151784 A1 offenbart ein Visualisierungssystem mit einem Operationsmikroskop und mit einem Endoskop, bei dem durch Betätigung eines Schalters an dem Endoskop ein auf das Endoskop abgestimmter Betriebszustand des Operationsmikroskops eingestellt werden kann.

In der US 6,398,721 B1 ist ein Operationsmikroskop beschrieben, das eine Mikroskopeinheit hat, die an einem Stativ aufgenommen ist und die optische Baugruppen für das Beobachten eines Operationsbereichs unter Vergrößerung mit einem optischen Beobachtungsstrahlengang enthält. Das Visualisierungssystem weist eine endoskopische Untersuchungseinrichtung auf, die als ein Video-Endoskop ausgebildet sein kann, das sich über in der Mikroskopeinheit ausgebildete elektrische Kontakte mit dem Operationsmikroskop verbinden lässt.

Die US 2005/0020876 A1 und die US 2001/055062 derselben Patentfamilie offenbaren jeweils ein Visualisierungssystem mit einem Operationsmikroskop, das eine Mikroskopeinheit hat, in der endoskopische Bilddaten zur Anzeige gebracht werden können. Für das Erfassen der endoskopischen Bilddaten gibt es in diesem Visualisierungssystem ein an einer Stativeinrichtung aufgenommenes Endoskop. An dem Stativ kann die Lage des Endoskops zu dem Mikroskop referenziert werden. Dies ermöglicht ein orts- und lagerichtiges Visualisieren von Endoskopbildern in dem Beobachtungsbild des Operationsmikroskops.

Aufgabe der Erfindung ist es, ein Visualisierungssystem bereitzustellen und ein Verfahren für den Betrieb eines chirurgischen Visualisierungssystems anzugeben, mit dem einer Beobachtungsperson in einer chirurgischen Operation auf ergonomisch günstige Weise das Anzeigen endoskopischer Bilder ermöglicht wird.

Diese Aufgabe wird mit dem in Anspruch 1 angegebenen Visualisierungssystem gelöst. Vorteilhafte Ausführungsformen der Erfindung sind in den abhängigen Ansprüchen angegeben.

Unter einem Operationsmikroskop wird vorliegend ein System mit einer vorzugsweise als ein Stereomikroskop ausgebildeten Mikroskopeinheit verstanden, die an einem Stativ aufgenommen ist und die einer Beobachtungsperson das Beobachten eines Operationsbereichs mit Vergrößerung ermöglicht. Die Mikroskopeinheit kann für das Visualisieren des Operationsbereichs mit einem optischen Beobachtungsstrahlengang ausgelegt sein. Es ist jedoch auch möglich, eine Mikroskopeinheit vorzusehen, die einer Beobachtungsperson digital erfasste Bilder zur Anzeige bringt. Ein Beispiel für ein Operationsmikroskop im Sinne der Erfindung ist etwa das von der Carl Zeiss Meditec AG hergestellte und vertriebene System OPMI^{®} Pentero^{®}.

Ein Endoskop im Sinne der Erfindung ist ein optisches Instrument zur Betrachtung und Untersuchung von Körperhöhlen. Endoskope im Sinne der Erfindung haben einen vorzugsweise in eine Längsrichtung erstreckten Endoskopkörper. Unter einem Video-Endoskop versteht die Erfindung ein Endoskop, welches das digitale Visualisieren von Körperhöhlen ermöglicht. Video-Endoskope enthalten eine Einrichtung für das Abbilden eines Operationsbereichs auf einem Bildsensor. Endoskopische Bilddaten im Sinne dieser Erfindung sind Bilddaten, die mit einem Video-Endoskop erfasst sind.

Die Erfindung schlägt vor, dass das Visualisierungssystem einen für das Erfassen einer Betätigungsinformation ausgelegten und von einer Beobachtungsperson betätigbaren Schaltkreis enthält, der bei Vorliegen der Betätigungsinformation für das Operationsmikroskop einen auf die Einrichtung für das Erfassen von endoskopischen Bildern in dem Operationsbereich abgestimmten Operationsmikroskop-Betriebszustand einstellt.

Der Schaltkreis kann z. B. vollständig oder teilweise in der Einrichtung für das Erfassen von endoskopischen Bildern in dem Operationsbereich angeordnet sein.

Ein auf die Einrichtung für das Erfassen von endoskopischen Bildern in dem Operationsbereich abgestimmter Operationsmikroskop-Betriebszustand kann z. B. in einer bestimmten Einstellung eines Systems für das Einstellen der Vergrößerung des Beobachtungsbildes des Operationsmikroskops (Vergrößerungssystem), in einer bestimmten Einstellung eines Beleuchtungssystems, das Beleuchtungslicht für das Beleuchten des Objektbereichs des Operationsmikroskops bereitstellt, oder in einer bestimmten Einstellung von Filtern in einem Beleuchtungsstrahlengang und/oder in einem Beobachtungsstrahlengang des Operationsmikroskops oder auch in einer bestimmten Konfiguration eines Displays des Operationsmikroskops bestehen.

Der Schaltkreis kann dabei ein Aktivierungsschaltkreis sein, der die Einrichtung für das Erfassen der endoskopischen Bilder aus einem Ruhezustand in einen Aktivierungszustand versetzt und der einen Aktivierungssensor enthält. Eine Idee der Erfindung ist es dabei, den Aktivierungssensor als einen Sensor aus der Gruppe Gyrosensor, Hallsensor, Berührungssensor oder Sprachsensor auszubilden.

Erfindungsgemäß wird vorgeschlagen, dass die Einrichtung für das Erfassen von endoskopischen Bildern in dem Operationsbereich wenigstens ein Endoskop aufweist. In diesem Fall ist der Aktivierungssensor bevorzugt in einem Endoskopkörper des Endoskops angeordnet. Es sei allerdings bemerkt, dass der Aktivierungssensor auch in dem Operationsmikroskop selbst angeordnet sein kann.

Eine Idee der Erfindung ist es, dass die Einrichtung für das Erfassen von endoskopischen Bildern in dem Operationsbereich wenigstens ein Endoskop enthält, das in einem ersten Betriebszustand das Untersuchen von in dem Operationsbereich gestreuten Weißlicht und das in einem von dem ersten Betriebszustand verschiedenen weiteren Endoskop-Betriebszustand das Untersuchen von Fluoreszenzlicht in einem definierten Wellenlängenbereich eines zu Fluoreszenz angeregten Farbstoffs wie etwa 5-ALA, Natriumfluorscein (NaFl), oder auch Indocyaningrün (ICG) in dem Operationsbereich und/oder das Untersuchen von Autofluoreszenzlicht in dem definierten Wellenlängenbereich von biologischem Gewebe und/oder Objekten in dem Operationsbereich ermöglicht.

Der Farbstoff 5-ALA wird z. B. mit Licht der Wellenlänge 400nm ≤ λ ≤ 410 nm zu Fluoreszenz angeregt. Für die Wellenlänge λ des Fluoreszenzlichts gilt dann 620 ≤ λ ≤ 710 nm. Der Farbstoff NaFI wird mit Licht der Wellenlänge 460nm ≤ λ ≤ 500 nm zu Fluoreszenz angeregt. Dabei entsteht Fluoreszenzlicht in dem Wellenlängenbereich 540 ≤ λ ≤ 690 nm. Für die Fluoreszenzanregung des Farbstoffs ICG muss dieser mit Licht der Wellenlänge 700nm ≤ λ ≤ 780nm beaufschlagt werden. Die Wellenlänge λ des ausgesendeten Fluoreszenzlichts liegt dann in dem Wellenlängenbereich 820nm ≤ λ ≤ 900 nm.

Eine Idee der Erfindung ist, dass das Operationsmikroskop in einem ersten Operationsmikroskop-Betriebszustand das Untersuchen von in dem Operationsbereich gestreuten Weißlicht und das in einem von dem ersten Betriebszustand verschiedenen weiteren Operationsmikroskop-Betriebszustand das Untersuchen von Fluoreszenzlicht in einem definierten Wellenlängenbereich eines zu Fluoreszenz angeregten ersten Farbstoffs, z. B. 5-ALA in dem Operationsbereich und/oder das Untersuchen einer Autofluoreszenz in dem definierten Wellenlängenbereich von biologischem Gewebe und/oder Objekten in dem Operationsbereich ermöglicht. Dabei ist das Endoskop erfindungsgemäß so ausgelegt, dass in einem ersten Endoskop-Betriebszustand das Untersuchen von in dem Operationsbereich gestreutem Weißlicht und in einem von dem ersten Betriebszustand verschiedenen weiteren Endoskop-Betriebszustand das Untersuchen von Fluoreszenzlicht in dem definierten Wellenlängenbereich des zu Fluoreszenz angeregten ersten Farbstoffs, z. B. 5-ALA in dem Operationsbereich und/oder das Untersuchen von Autofluoreszenzlicht in dem definierten Wellenlängenbereich von biologischem Gewebe und/oder Objekten in dem Operationsbereich möglich ist. Der weitere Endoskop-Betriebszustand und der weitere Operationsmikroskop-Betriebszustand sind also aufeinander abgestimmt. Erfindungsgemäß sind das Operationsmikroskop und das Endoskop dabei derart wirkungsgekoppelt, dass bei Einstellen des weiteren Endoskop-Betriebszustands der weitere Operati-onsmikroskop-Betriebszustand automatisch, d. h. ausgelöst durch das Einstellen des weiteren Endoskop-Betriebszustands eingestellt wird.

Es ist außerdem eine Idee der Erfindung, in dem Visualisierungssystem eine Einrichtung für das automatische Wirkungskoppeln des Operationsmikroskops und dem ersten Endoskop bei einem Aktivieren des Endoskops und/oder bei einem Aufnehmen des Endoskops durch die Beobachtungsperson und/oder bei einem Anordnen eines Abschnitts des Endoskops in einem Beobachtungsbereich des Operationsmikroskops und/oder bei einem Auftreten von Fluoreszenzlicht und/oder Autofluoreszenzlicht in dem definierten Wellenlängenbereich vorzusehen.

Erfindungsgemäß wird auch vorgeschlagen, dass es in dem Visualisierungssystem ein weiteres Endoskop gibt, das in wenigstens einem Endoskop-Betriebszustand das Untersuchen von Fluoreszenzlicht in einem definierten weiteren Wellenlängenbereich eines zu Fluoreszenz angeregten weiteren Farbstoffs, z. B. NaFI in dem Operationsbereich und/oder das Untersuchen von Autofluoreszenzlicht in dem definierten weiteren Wellenlängenbereich von biologischem Gewebe und/oder Objekten in dem Operationsbereich ermöglicht, wobei das Operationsmikroskop in einem von dem ersten Betriebszustand verschiedenen weiteren Operationsmikroskop-Betriebszustand das Untersuchen von Fluoreszenzlicht in dem definierten Wellenlängenbereich des zu Fluoreszenz angeregten weiteren Farbstoffs, z. B. NaFI in dem Operationsbereich und/oder das Untersuchen einer Autofluoreszenz in dem definierten Wellenlängenbereich von biologischem Gewebe und/oder Objekten in dem Operationsbereich ermöglicht, und wobei das Operationsmikroskop und das Endoskop derart wirkungskoppelbar sind, dass bei einem Betrieben des Endoskops in diesen weiteren Endoskop-Betriebszustand der weitere Operationsmikroskop-Betriebszustand automatisch eingestellt wird. Eine Idee der Erfindung ist es dabei insbesondere, dass die Einrichtung für das automatische Wirkungskoppeln des Operationsmikroskops und des weiteren Endoskops bei einem Aktivieren des weiteren Endoskops und/oder bei einem Aufnehmen des weiteren Endoskops durch die Beobachtungsperson und/oder bei einem Anordnen eines Abschnitts des weiteren Endoskops in einem Beobachtungsbereich des Operationsmikroskops und/oder bei einem Auftreten von Fluoreszenzlicht und/oder Autofluoreszenzlicht in dem definierten Wellenlängenbereich automatisch, d. h. ausgelöst durch das Aufnehmen des weiteren Endoskops und/oder das Anordnen eines Abschnitts des weiteren Endoskops in dem Beobachtungsbereich und/oder durch das Auftreten von Fluoreszenzlicht und/oder Autofluoreszenzlicht bewirkt wird.

In dem Visualisierungssystem kann insbesondere auch ein weiteres Endoskop vorgesehen sein, das in wenigstens einem Endoskop-Betriebszustand das Untersuchen von Fluoreszenzlicht in einem definierten weiteren Wellenlängenbereich eines zu Fluoreszenz angeregten weiteren Farbstoffs z. B. ICG in dem Operationsbereich und/oder das Untersuchen von Autofluoreszenzlicht in dem definierten weiteren Wellenlängenbereich von biologischem Gewebe und/oder Objekten in dem Operationsbereich ermöglicht, wobei das Operationsmikroskop in einem von dem ersten Betriebszustand verschiedenen weiteren Operationsmikroskop-Betriebszustand das Untersuchen von Fluoreszenzlicht in dem definierten weiteren Wellenlängenbereich des zu Fluoreszenz angeregten weiteren Farbstoffs, z. B. ICG in dem Operationsbereich und/oder das Untersuchen einer Autofluoreszenz in dem definierten Wellenlängenbereich von biologischem Gewebe und/oder Objekten in dem Operationsbereich ermöglicht, und wobei das Operationsmikroskop und das Endoskop derart wirkungskoppelbar sind, dass bei einem Betreiben des Endoskops in diesem Endoskop-Betriebszustand der weitere Operationsmikroskop-Betriebszustand automatisch eingestellt wird.
Eine Idee der Erfindung ist hier wiederum das automatische Wirkungskoppeln des Operationsmikroskops und des weiteren Endoskops bei einem Aktivieren des weiteren Endoskops und/oder bei einem Aufnehmen des weiteren Endoskops durch die Beobachtungsperson und/oder bei einem Anordnen eines Abschnitts des weiteren Endoskops in einem Beobachtungsbereich des Operationsmikroskops und/oder bei einem Auftreten von Fluoreszenzlicht und/oder Autofluoreszenzlicht in dem definierten weiteren Wellenlängenbereich.
In dem erfindungsgemäßen Visualisierungssystem kann das wenigstens eine Endoskop insbesondere als ein Video-Endoskop ausgebildet sein.
Es wird darüber hinaus vorgeschlagen, dass das Visualisierungssystem auch eine Einrichtung für das Drehen eines an einem Display angezeigten endoskopischen Bilds aus dem Operationsbereich relativ zu dem Display hat.

Bei einem Verfahren zum Betrieb eines Visualisierungssystems, das ein Operationsmikroskop für das Beobachten eines Operationsbereichs mit Vergrößerung enthält und das eine Einrichtung für das Erfassen von endoskopischen Bilddaten in dem Operationsbereich aufweist, die für das Anzeigen der endoskopischen Bilddaten mit dem Operationsmikroskop wirkungsgekoppelt ist, wird bei einem Verstellen wenigstens eines Betriebsparameters des Video-Endoskops wenigstens ein Betriebsparameter des Operationsmikroskops verändert und/oder bei einem Verstellen wenigstens eines Betriebsparameters des Operationsmikroskops wenigstens einen Betriebsparameter des Video-Endoskops variiert.

Nachfolgend werden vorteilhafte Ausführungsbeispiele der Erfindung beschrieben, die in den Zeichnungen schematisch dargestellt sind.

Es zeigen:
- Fig. 1: ein erstes Visualisierungssystem mit einem Operationsmikroskop und mit einer Einrichtung für das Erfassen von endoskopischen Bilddaten mit einem ersten, mit einem zweiten und mit einem dritten Video-Endoskop;
- Fig. 2: das erste Video-Endoskop in der Einrichtung für das Erfassen von elektronischen Bilddaten als Schnitt;
- Fig.3: den Aufbau des Operationsmikroskops in dem chirurgischen Visualisierungssystem;
- Fig. 4: ein Filterrad eines Beleuchtungssystems in dem Operationsmikroskop;
- Fig. 5: ein in einem Beobachtungsstrahlengang des Operationsmikroskops angeordnetes Filterrad;
- Fig. 6a: und Fig. 6b sowie Fig. 6c und Fig. 6d verschiedene Anzeigen an einem Display des Operationsmikroskops; und
- Fig. 7: ein weiteres Visualisierungssystem mit einem Operationsmikroskop und mit einer Einrichtung für das Erfassen von endoskopischen Bilddaten mit einem ersten, mit einem zweiten und mit einem dritten Video-Endoskop.

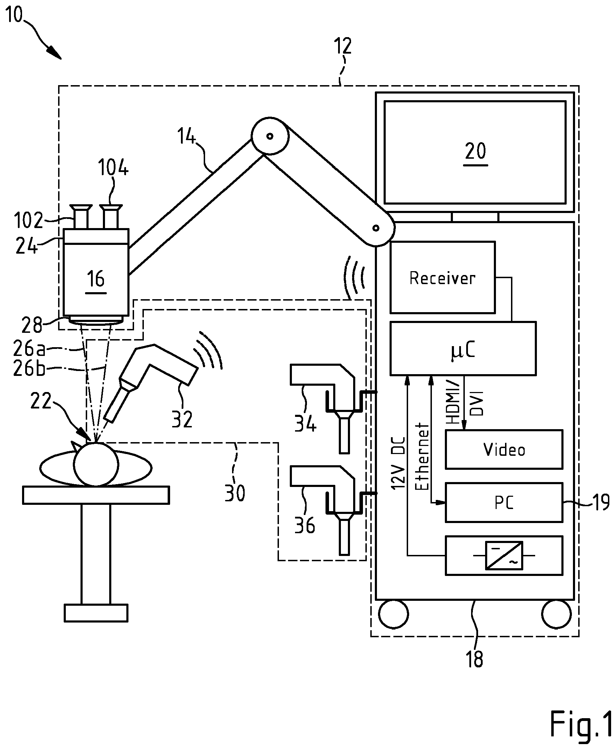

Das in der Fig. 1 gezeigte Visualisierungssystem 10 ist ein chirurgisches Visualisierungssystem. Es umfasst ein Operationsmikroskop 12 mit einer an einem Stativ 14 aufgenommenen Mikroskopeinheit 16 und mit einer Bedienkonsole 18, die eine Rechnereinheit 19 mit einem berührungssensitiven Display 20 enthält. Das Operationsmikroskop 12 ermöglicht einer Beobachtungsperson das stereoskopische Betrachten eines Operationsbereichs 22 in einem Binokulareinblick 24, der Okulare 102, 104 hat, mit einem linken und rechten optischen Beobachtungsstrahlengang 26a, 26b, der ein gemeinsames Mikroskop-Hauptobjektiv 28 durchsetzt.

In dem Visualisierungssystem 10 gib es eine Einrichtung 30 für das Erfassen von endoskopischen Bilddaten. Die Einrichtung 30 enthält ein erstes, ein zweites und ein drittes Video-Endoskop 32, 34, 36. Das Video-Endoskop 32 ermöglicht das Untersuchen des Operationsbereichs 22 durch Erfassen von in dem Operationsbereich 22 gestreuten Weißlicht und des Fluoreszenzlichts des zu Fluoreszenz angeregten Farbstoffs 5-ALA in dem Operationsbereich 22.

Das Video-Endoskop 34 dient für das Untersuchen des Operationsbereichs 22 durch Erfassen von in dem Operationsbereich 22 gestreuten Weißlicht und des Fluoreszenzlichts des zu Fluoreszenz angeregten Farbstoffs Natriumfluoreszein (NaFI).

Das Video-Endoskop 36 eignet sich für das Untersuchen des Operationsbereichs 22 durch Erfassen von in dem Operationsbereich 22 gestreuten Weißlicht und des Fluoreszenzlichts des zu Fluoreszenz angeregten Farbstoffs IndiocyaninGrün (ICG) in dem Operationsbereich 22.

Das berührungssensitive Display 20 des Operationsmikroskops 12 ermöglicht zum einen das Steuern und Einstellen von optischen Abbildungsparametern der Mikroskopeinheit 16 sowie der Video-Endoskope 32, 34 und 36 und zum anderen das getrennte oder zeitgleiche Visualisieren des mittels eines Video-Endoskops 32, 34, 36 oder mittels der Mikroskopeinheit 16 beobachteten Objektbereichs 22.

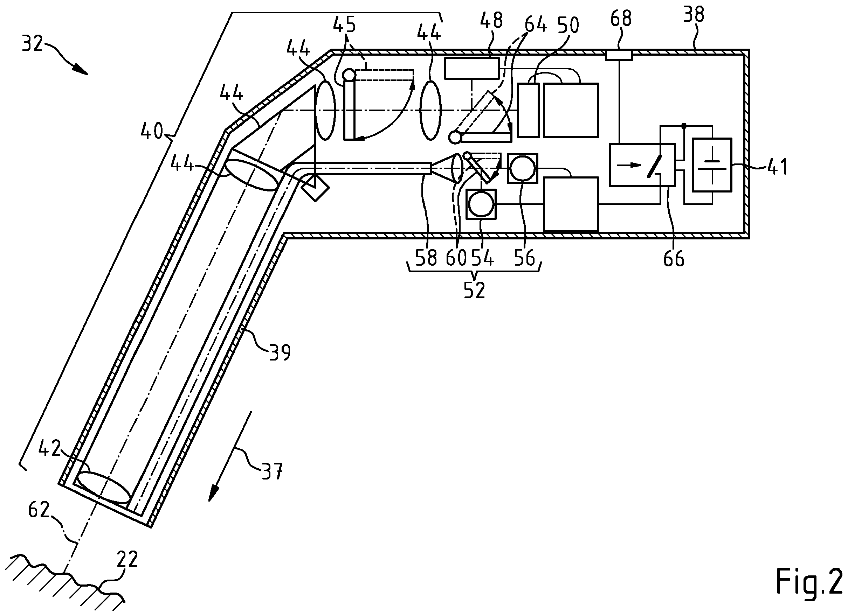

Die Fig. 2 zeigt den Aufbau des ersten Video-Endoskops 32 aus Fig. 1. Das Video-Endoskop 32 hat einen in die Längsrichtung 37 erstreckten Endoskopkörper 39 mit einem daran angeschlossenen Griffstück 38, in dem ein elektrischer Energiespeicher 41 angeordnet ist. Der elektrische Energiespeicher 41 ist eine Batterie. Grundsätzlich ist es allerdings auch möglich, einen Hochleistungs-Kondensator als elektrischen Energiespeicher 41 vorzusehen. Das Video-Endoskop 32 ermöglicht das Beobachten des Objektbereichs 46 in einem Weißlichtbetriebsmodus und in einem Fluoreszenzlichtbetriebsmodus. Hierfür enthält das Video-Endoskop 32 eine Beobachtungsoptik 40, die eine Objektivbaugruppe 42 aufweist und ein optisches Übertragungssystem 44 mit einem Klappspiegel 45 umfasst, um ein Bild eines Operationsbereichs 22 wahlweise einem für Infrarotlicht sensitiven Bildsensor 48 und einem für Licht im sichtbaren Spektralbereich sensitiven Bildsensor 50 zuzuführen.

Um den Operationsbereich 22 zu beleuchten, gibt es in dem Video-Endoskop 32 ein Beleuchtungssystem 52 mit einer Weißlicht-LED 54 und mit einer Lichtquelle 56, die Licht bereitstellt, mit dem der Fluoreszenzfarbstoff schmalbandig zu Fluoreszenz angeregt werden kann. Das Beleuchtungssystem 52 enthält hierfür einen Lichtleiter 58 und weist einen schaltbaren Klappspiegel 60 auf, der es ermöglicht, den Objektbereich 46 wahlweise mit dem Licht der Weißlicht-LED 54 oder der Lichtquelle 56 zu beleuchten. Um das von fluoreszierenden Objekten, z. B. dem Farbstoff ICG, ausgehende Fluoreszenzlicht, das in dem Operationsbereich 22 gestreut wird und das durch die Objektivbaugruppe 42 in das Übertragungssystem 44 gelangt, unterdrücken zu können, gibt es in dem Video-Endoskop 32 ein schaltbares Emissionsfilter 64, das wahlweise in und aus dem optischen Abbildungsstrahlengang 62 bewegt werden kann.

Das Video-Endoskop 32 enthält einen Aktivierungsschaltkreis 66, der in dem Endoskopkörper 39 angeordnet ist. Der Aktivierungsschaltkreis 66 weist als Aktivierungssensor einen in das Griffstück 38 integrierten Berührungssensor 68 auf, mit dem das Aufnehmen des Video-Endoskops 32 an dem Griffstück 38 mit der Hand einer Beobachtungsperson erfasst werden kann, um das Video-Endoskop 32 dann aus einem Ruhemodus in einen Arbeitsmodus zu versetzen. Der Aktivierungsschaltkreis 66 bewirkt hier ein automatisches wirkungskoppeln des Video-Endoskops 32 und des Operationsmikroskops 12. Das Video-Endoskop 32 wird mittels des Aktivierungsschaltkreises 66 an der Rechnereinheit 19 des Operationsmikroskops 12 angemeldet. Es übermittelt dann an die Rechnereinheit 19 drahtlos elektronische Bilddaten, die an dem berührungssensitiven Display 20 des Operationsmikroskops 12 angezeigt werden können.

Wenn eine Beobachtungsperson das Griffstück 38 mit der Hand freigibt, wird dies mittels des Berührungssensors 68 erfasst. Der Aktivierungsschaltkreis 66 versetzt dann das Video-Endoskop 32 wieder in den Ruhemodus. Auf diese Weise kann der elektrische Energieverbrauch des Video-Endoskops 32 minimiert werden, wenn dieses lediglich bereitgestellt, nicht aber benutzt wird.

Der Aufbau des zweiten Video-Endoskops 34 und des dritten Video-Endoskops 36 aus Fig. 1 entspricht grundsätzlich dem Aufbau des ersten Video-Endoskops 32. Das zweite Video-Endoskop 34 und das dritte Video-Endoskop 36 enthalten dabei allerdings neben der Weißlicht-LED jeweils eine Lichtquelle und ein Emissionsfilter, die für das Anregen und Detektieren von Fluoreszenzlicht des Farbstoffs 5-ALA bzw. NaFI abgestimmt sind. Auch hier gibt es einen Aktivierungsschaltkreis mit einem Berührungssensor, der die vorstehend beschriebene Funktionalität hat.

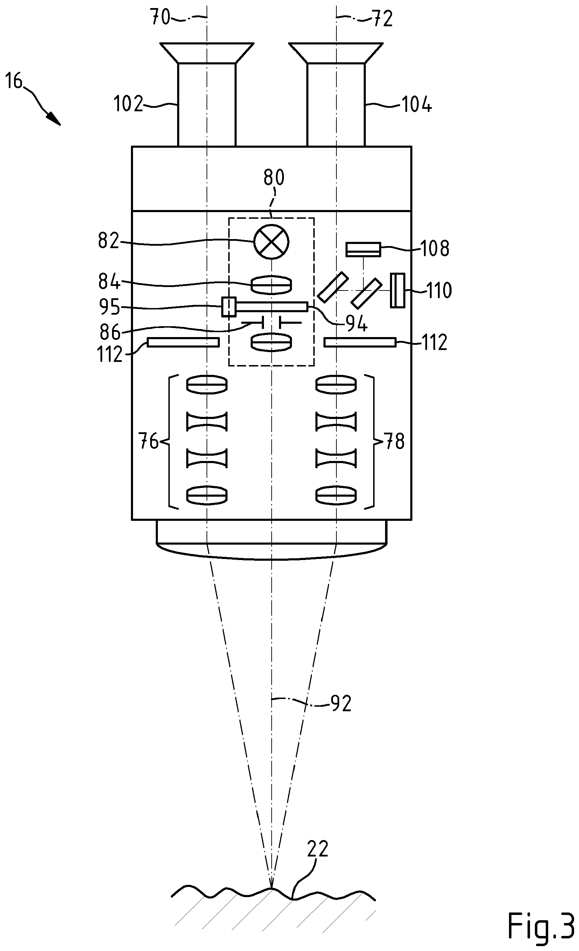

Die Fig. 3 erläutert den Aufbau der Mikroskopeinheit 16 des Operationsmikroskops 12 in dem Visualisierungssystem 10. Die Mikroskopeinheit 16 ermöglicht das stereoskopische Beobachten des Operationsbereichs 22 mit Beobachtungsstrahlengängen 70, 72, die ein Mikroskop-Hauptobjektiv 74 durchsetzen. In der Mikroskopeinheit 16 ist zu einer Einstellung der Vergrößerung in den Beobachtungsstrahlengängen 70, 72 ein Zoomsystem 76, 78 vorgesehen. Für das Beleuchten des Operationsbereichs 22 mit Beleuchtungslicht gibt es in der Mikroskopeinheit 16 ein Beleuchtungssystem 80 mit einer Lichtquelle 82.

Das von der Lichtquelle 82 ausgesendete Licht wird mit einer Kollimationsoptik 84 in der Ebene einer Leuchtfeldblende 86 kollimiert und über die Kondensorlinse 88 und das Mikroskop-Hauptobjektiv 28 mit dem Beleuchtungsstrahlengang 92 in den Operationsbereich 22 geführt. Das Beleuchtungssystem 80 enthält ein verstellbares Filterrad 94, das mittels eines Antriebs 95 verstellbar ist und das unterschiedliche Filter für das Einstellen der spektralen Zusammensetzung des zu dem Operationsbereich 22 geführten Beleuchtungslichts aufweist.

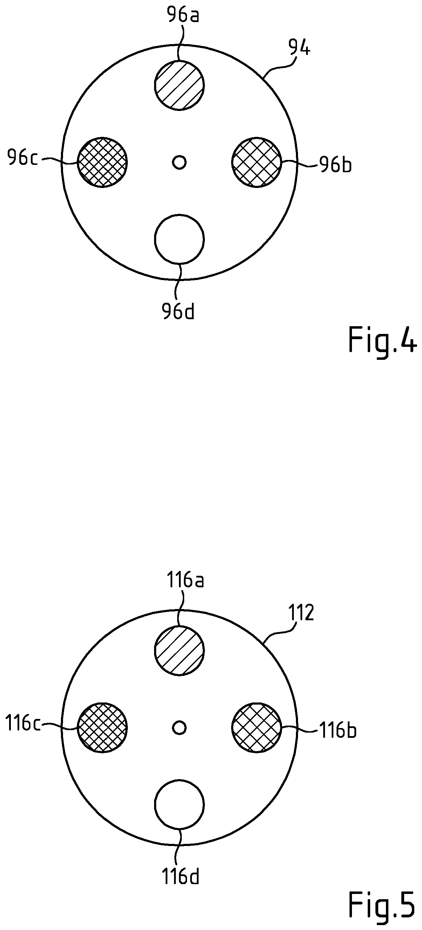

Die Fig. 4 zeigt das Filterrad 94 als Draufsicht. Das Filterrad 94 hat eine Lochblende 96d und Beleuchtungsfilter 96a, 96b, 96c, mit denen die spektrale Zusammensetzung des Beleuchtungslichts so eingestellt werden kann, dass sich damit der Farbstoff ICG oder 5-ALA bzw. NaFI zu Fluoreszenz anregen lässt und das Licht der Wellenlänge, das der Wellenlänge des Fluoreszenzlichts dieser Farbstoffe entspricht, dabei ausgefiltert ist.

In dem linken und rechten Beobachtungsstrahlengang 70, 72 der Mikroskopeinheit 16 gibt es jeweils ein verstellbares Filterrad 112. Die Fig. 5 zeigt das Filterrad 112 als Draufsicht. Das Filterrad 112 enthält die Filter 116a, 116b, 116c und eine Lochblende 116d. Die Transmissionscharakteristik der Filter 116a, 116b, 116c ist auf die Transmissionscharakteristik der Beleuchtungsfilter 96a, 96b, 96c abgestimmt. Mittels der Filter 116a, 116b, 116c wird das die Fluoreszenz des Farbstoffs ICG oder 5-ALA bzw. NaFI anregende Licht unterdrückt und das Licht der Wellenlänge des Fluoreszenzlichts dieser Farbstoffe hindurchgelassen.

Um in dem Operationsbereich 22 mittels Fluoreszenzlicht Gewebestrukturen zu visualisieren, die mit dem Farbstoff 5-ALA angereichert sind, wird das Filter 96a des Filterrads 94 in den Beleuchtungsstrahlengang 92 geschaltet und das Filter 116a des Filterrads 112 in dem linken und rechten Beobachtungsstrahlengang 70, 72 angeordnet. Entsprechend wird für das Visualisieren von mit dem Farbstoff ICG angereicherten Gewebestrukturen in dem Operationsbereich 22 das Filter 96b in dem Beleuchtungsstrahlengang 92 angeordnet und das Filter 116b des entsprechenden Filterrads 112 in dem linken bzw. rechten Beobachtungsstrahlengang 70, 72 positioniert. Für das Visualisieren von Gewebestrukturen, die den Farbstoff NaFI enthalten, wird das Filter 96c in den Beleuchtungsstrahlengang 92 geschaltet und das Filter 116c des Filterrads 112 in dem linken und rechten Beobachtungsstrahlengang 70, 72 positioniert.

In der Mikroskopeinheit 16 gibt es eine Kamera 108 für das Erfassen von IR-Licht und eine Kamera 110, mit der Fluoreszenzlicht in dem sichtbaren Spektralbereich erfasst werden kann. Die Kamera 108 und die Kamera 110 ist mit der in der Fig. 1 gezeigten Rechnereinheit 19 des Operationsmikroskops 12 verbunden.

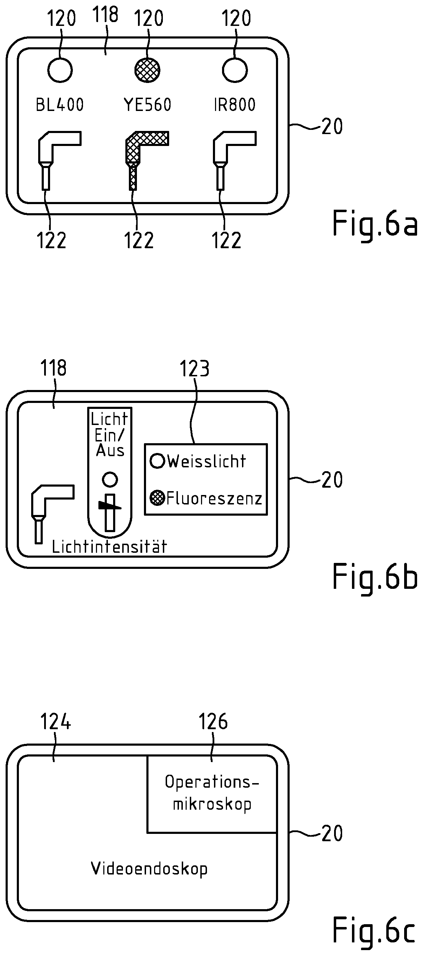

Die Fig. 6a zeigt eine durch das Aktivieren eines Video-Endoskops 32, 34, 36 ausgelöste Anzeige 118 des Displays 20. Eine Beobachtungsperson wird damit informiert, mit welchem der Video-Endoskope sie gerade arbeitet.

In der Fig. 6b ist eine Anzeige 118 des Displays 20 mit Schaltflächen für das Steuern eines aktivierten Video-Endoskops 32, 34, 36 gezeigt. Die Schaltfläche 123 ermöglicht einer Bedienperson das Umschalten des entsprechend aktivierten Video-Endoskops 32, 34, 36 zwischen dem Betriebsmodus für das Beobachten des Operationsbereichs 22 mittels Weißlicht und dem Betriebsmodus für das Beobachten des Operationsbereichs 22 durch Erfassen von Fluoreszenzlicht. Dabei wird bei einem Umschalten eines Video-Endoskops 32, 34, 36 zwischen dem Weißlichtbetriebsmodus und dem Fluoreszenzlichtbetriebsmodus mittels des entsprechenden Aktivierungsschaltkreises auch der Betriebsmodus der Mikroskopeinheit 16 von einem Weißlichtbetriebsmodus in den Fluoreszenzlichtbetriebsmodus und umgekehrt geschaltet. Diese Maßnahme bewirkt, dass in dem Visualisierungssystem 10 der Betriebszustand des Operationsmikroskops 12 auf den Betriebszustand des eingesetzten Video-Endoskops 32, 34, 36 immer automatisch abgestimmt ist.

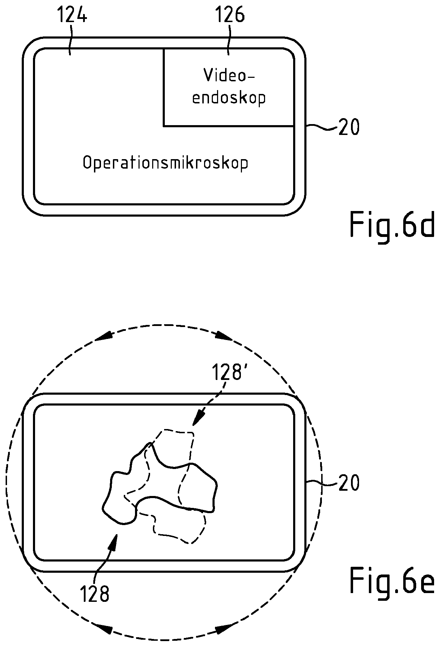

Die Fig. 6c und Fig. 6d zeigen jeweils ein Beobachtungsbild eines Video-Endoskops 32, 34, 36 und ein Beobachtungsbild der Mikroskopeinheit 16 an dem Display 20. Hier wird das Beobachtungsbild des Video-Endoskops 32, 34, 36 und der Mikroskopeinheit 16 in zwei voneinander getrennten Feldern 124, 126 visualisiert, die unterschiedliche Abmessungen haben. Durch Berühren des jeweils kleineren Feldes 126 kann eine Beobachtungsperson hier in dem größeren Feld 124 wahlweise das Beobachtungsbild der Mikroskopeinheit 16 oder des ausgewählten Video-Endoskops 32, 34, 36 zur Anzeige bringen.

Optional kann bei dem Visualisierungssystem auch vorgesehen sein, dass ausgelöst durch das Aufnehmen oder Einschalten eines bestimmten Video-Endoskops 32, 34, 36 durch eine Beobachtungsperson das Beobachtungsbild des entsprechenden Video-Endoskops 32, 34, 36 in dem größeren Feld 124 automatisch zur Anzeige gebracht wird. In diesem Fall ist also der Aktivierungssensor 68 für ein Video-Endoskop 32, 34, 36 in das Operationsmikroskop 12 integriert und es befindet sich der Aktivierungsschaltkreis 66 jeweils teilweise in dem Operationsmikroskop 12 und in einem Video-Endoskop 32, 34, 36.

Die Fig. 6e zeigt das Beobachtungsbild des Video-Endoskops 32, 34, 36 als ein Vollbild 128. Eine Beobachtungsperson kann dieses Vollbild 128 drehen, indem sie mit den Fingern einer Hand einer Drehrichtung folgend über das Display 20 wischt oder indem sie eine gewünschte Drehposition des Bilds 128, 128' mit den Fingern einer Hand an dem Display 20 antippt.

Es sei bemerkt, dass ein Video-Endoskop 32, 34, 36 in einer alternativen Ausführungsform des chirurgischen Visualisierungssystems in dem Aktivierungsschaltkreis 66 als Aktivierungssensor anstelle eines Berührungssensors auch einen Gyrosensor aufweisen kann, der bei Erfassen einer Verlagerung des entsprechenden Video-Endoskops 32, 34, 36 dieses in einen Arbeitszustand schaltet. Alternativ hierzu ist es auch möglich, in dem Aktivierungsschaltkreis einen Hallsensor vorzusehen, mit dem erfasst werden kann, ob das entsprechende Video-Endoskop 32, 34, 36 aus einer Ruheposition bewegt wird, in der das Video-Endoskop 32, 34, 36 in dem Abschnitt des Hallsensors einem definierten Magnetfeld ausgesetzt ist. Außerdem sei bemerkt, dass anstelle der vorgenannten Sensoren in dem Aktivierungsschaltkreis 66 als Aktivierungssensor auch ein Sprachsensor vorgesehen sein kann, um ein Video-Endoskop aus einem Ruhezustand in einen Arbeitszustand zu schalten.

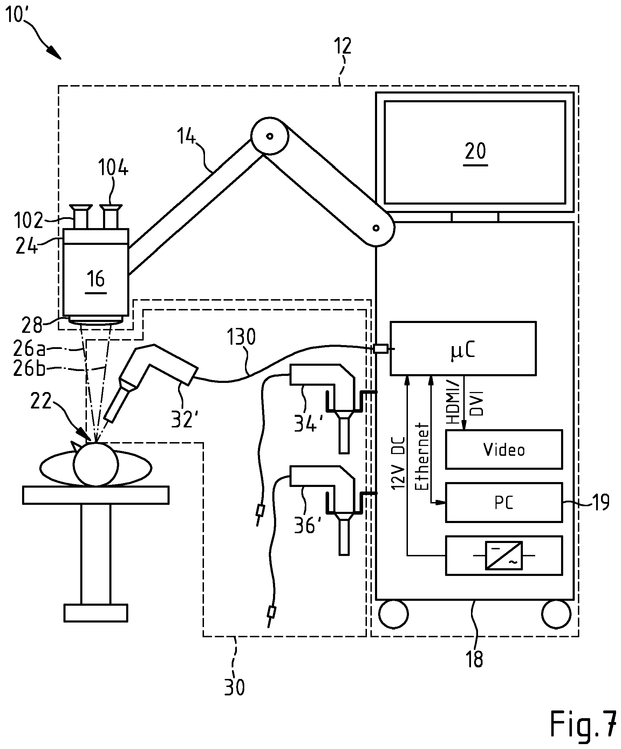

Die Fig. 7 zeigt ein weiteres Visualisierungssystem 10' mit einem Operationsmikroskop 12 und mit einer Einrichtung für das Erfassen von elektronischen Bilddaten mit einem ersten, einem zweiten sowie einem dritten Video-Endoskop 32', 34', 36'. Soweit Baugruppen des Visualisierungssystems 10' und Elemente in der Fig. 5 Elementen und Baugruppen aus den vorstehenden Figuren entsprechen, sind diese hier mit den gleichen Zahlen als Bezugszeichen kenntlich gemacht. Für den Anschluss der Video-Endoskope 32', 34', 36' an das Operationsmikroskop 12 ist hier ein Kabel 130 mit einem Stecker 132 und einer in der Bedienkonsole 18 des Operationsmikroskops 12 ausgebildete Steckdose 134 vorgesehen. Das Einstecken des Steckers 132 in die Steckdose 134 betätigt hier einen in dem entsprechenden Video-Endoskop 32', 34', 36' angeordneten Aktivierungsschaltkreis, der bei Aktivierung das Anmelden des entsprechenden Video-Endoskops 32', 34', 36' an der Rechnereinheit 19 des Operationsmikroskops 12 bewirkt und für das entsprechende Video-Endoskop 32', 34', 36' einen Arbeitsbetriebsmodus einstellt. Diese Maßnahme hat dann das Anzeigen der Betriebsparameter des entsprechenden Video-Endoskops 32', 34', 36' an dem Display 20 zur Folge und ermöglicht so das Anzeigen von dessen Beobachtungsbild. Umgekehrt, wenn die Steckerverbindung von Video-Endoskop 32', 34', 36' und Operationsmikroskop 12 geöffnet wird, hat dies ein Abmelden des entsprechenden Video-Endoskops 32', 34', 36' an dem Operationsmikroskop 12 zur Folge und das entsprechende Video-Endoskop wird in einen Ruhemodus versetzt.

Zusammenfassend ist insbesondere folgendes festzuhalten: Die Beschreibung betrifft ein Visualisierungssystem 10, 10' mit einem Operationsmikroskop 12 für das Beobachten eines Operationsbereichs 22 mit Vergrößerung, das eine Rechnereinheit 19 mit einem Display 20 zum Anzeigen von Bilddaten aufweist. Das Visualisierungssystem 10, 10' umfasst eine Einrichtung 30 für das Erfassen von endoskopischen Bilddaten in dem Operationsbereich 22, die für das Anzeigen der endoskopischen Bilddaten mit dem Operationsmikroskop 12 wirkungsgekoppelt ist. Die Einrichtung 30 für das Erfassen von endoskopischen Bildern in dem Operationsbereich 22 enthält einen für das Erfassen einer Betätigungsinformation ausgelegten und von einer Beobachtungsperson betätigbaren Schaltkreis 66, der bei Vorliegen der Betätigungsinformation für das Operationsmikroskop 12 einen auf die Einrichtung 30 für das Erfassen von endoskopischen Bildern in dem Operationsbereich 22 abgestimmten Operationsmikroskop-Betriebszustand einstellt.

### Bezugszeichenliste

- 10, 10': Visualisierungssystem
- 12: Operationsmikroskop
- 14: Stativ
- 16: Mikroskopeinheit
- 18: Bedienkonsole
- 19: Rechnereinheit
- 20: Display
- 22: Operationsbereich
- 24: Binokulareinblick
- 26a, 26b: Beobachtungsstrahlengang
- 28: Mikroskop-Hauptobjektiv
- 30: Einrichtung zum Erfassen endoskopischer Bilddaten
- 32, 32', 32", 34, 34, 36, 36': Video-Endoskop
- 37: Längsrichtung
- 38: Griffstück
- 39: Endoskopkörper
- 40: Beobachtungsoptik
- 41: elektrischer Energiespeicher
- 42: Objektivbaugruppe
- 44: optisches Übertragungssystem
- 45: Klappspiegel
- 46: Objektbereich
- 48, 50: Bildsensor
- 52: Beleuchtungssystem
- 54: Weißlicht-LED
- 56: Lichtquelle
- 58: Lichtleiter
- 60: Klappspiegel
- 62: Abbildungsstrahlengang
- 64: Emissionsfilter
- 66: Aktivierungsschaltkreis
- 68: Berührungssensor
- 70, 72: Beobachtungsstrahlengang
- 74: Mikroskop-Hauptobjektiv
- 76, 78: Zoomsystem
- 80: Beleuchtungssystem
- 82: Lichtquelle
- 84: Kollimationsoptik
- 86: Leuchtfeldblende
- 88: Kondensorlinse
- 92: Beleuchtungsstrahlengang
- 94: Filterrad
- 95: Antrieb
- 96a, 96b, 96c: Filter
- 96d: Blende
- 102, 104: Okular
- 108, 110: Kamera
- 112: Filterrad
- 116a, 116b, 116c: Filter
- 116d: Lochblende
- 118: Anzeige
- 123: Schaltfläche
- 124, 126: Feld
- 128, 128': Bild
- 130: Kabel
- 132: Stecker
- 134: Steckdose

## Patentansprüche

1. Visualisierungssystem (10, 10')
mit einem Operationsmikroskop (12) eingerichtet zum Beobachten eines Operationsbereichs (22) mit Vergrößerung, das eine Rechnereinheit (19) mit einem Display (20) eingerichtet zum Anzeigen von Bilddaten enthält; und
mit einer Einrichtung (30) eingerichtet zum Erfassen von endoskopischen Bildern, die mit dem Operationsmikroskop (12) wirkungsgekoppelt ist und die wenigstens ein Endoskop (32, 32', 32") aufweist;
und mit einem für das Erfassen einer Betätigungsinformation ausgelegten und von einer Beobachtungsperson betätigbaren Schaltkreis (66), der bei Vorliegen der Betätigungsinformation für das Operationsmikroskop (12) einen auf die Einrichtung (30) für das Erfassen von endoskopischen Bildern in dem Operationsbereich (22) abgestimmten Operationsmikroskop-Betriebszustand einstellt;
**dadurch gekennzeichnet, dass**
das Operationsmikroskop (12) in einem ersten Operationsmikroskop-Betriebszustand zum Untersuchen von in dem Operationsbereich (22) gestreuten Weißlicht eingerichtet ist, und das in einem von dem ersten Betriebszustand verschiedenen weiteren Operationsmikroskop-Betriebszustand zum Untersuchen von Fluoreszenzlicht in einem definierten Wellenlängenbereich eines zu Fluoreszenz angeregten ersten Farbstoffs (5-ALA) in dem Operationsbereich (22) und/oder das Untersuchen einer Autofluoreszenz in dem definierten Wellenlängenbereich von biologischem Gewebe und/oder Objekten in dem Operationsbereich (22) eingerichtet ist, wobei das Endoskop (32, 32', 34, 34' 36, 36') in einem Endoskop-Betriebszustand zum Untersuchen von Fluoreszenzlicht in dem definierten Wellenlängenbereich des zu Fluoreszenz angeregten ersten Farbstoffs (5-ALA) in dem Operationsbereich (22) und/oder das Untersuchen von Autofluoreszenzlicht in dem definierten Wellenlängenbereich von biologischem Gewebe in dem Operationsbereich (22) eingerichtet ist, und wobei das Operationsmikroskop (12) und das Endoskop (32, 32', 34, 34' 36, 36') derart wirkungsgekoppelt sind, dass bei einem Betreiben des Endoskops (32, 32', 34, 34', 36, 36') in diesem Endoskop-Betriebszustand der weitere Operationsmikroskop-Betriebszustand automatisch eingestellt wird.

2. Visualisierungssystem nach Anspruch 1, **dadurch gekennzeichnet, dass** der Schaltkreis ein Aktivierungsschaltkreis (66) ist, der die Einrichtung für das Erfassen der endoskopischen Bilder aus einem Ruhezustand in einen Aktivierungszustand versetzt, wobei der Aktivierungsschaltkreis (66) einen Aktivierungssensor aus der Gruppe Gyrosensor, Hallsensor, Berührungssensor (68) oder Sprachsensor aufweist.

3. Visualisierungssystem nach Anspruch 2 oder 3, **dadurch gekennzeichnet, dass** das Endoskop (32, 32', 32") einen Endoskopkörper (39) hat, in dem der Aktivierungssensor (66) angeordnet ist.

4. Visualisierungssystem nach Anspruch 2, **dadurch gekennzeichnet, dass** der Aktivierungssensor in dem Operationsmikroskop (12) angeordnet ist.

5. Visualisierungssystem nach einem der Ansprüche 1 bis 4, **dadurch gekennzeichnet, dass** das wenigstens eine Endoskop (32, 32') in einem ersten Endoskop-Betriebszustand zum Untersuchen von in dem Operationsbereich (22) gestreuten Weißlicht und in einem von dem ersten Endoskop-Betriebszustand verschiedenen weiteren Endoskop-Betriebszustand zum Untersuchen von Fluoreszenzlicht in einem definierten Wellenlängenbereich eines zu Fluoreszenz angeregten Farbstoffs (5-ALA, NaFI, ICG) in dem Operationsbereich (22) und/oder zum Untersuchen von Autofluoreszenzlicht in dem definierten Wellenlängenbereich von biologischem Gewebe und/oder Objekten in dem Operationsbereich (22) eingerichtet ist.

6. Visualisierungssystem nach einem der Ansprüche 1 bis 5, **gekennzeichnet durch** eine Einrichtung eingerichtet für das automatische Wirkungskoppeln des Operationsmikroskops (12) mit dem wenigstens einen Endoskop (32, 32') bei einem Aktivieren des Endoskops (32, 32', 34, 34' 36, 36') und/oder bei einem Aufnehmen des Endoskops (32, 32', 34, 34' 36, 36') durch die Beobachtungsperson und/oder bei einem Anordnen eines Abschnitts des Endoskops (32, 32', 34, 34' 36, 36') in einem Beobachtungsbereich des Operationsmikroskops (12) und/oder bei einem Auftreten von Fluoreszenzlicht und/oder Autofluoreszenzlicht in dem definierten Wellenlängenbereich.

7. Visualisierungssystem nach einem der Ansprüche 1 bis 6, **gekennzeichnet durch** ein weiteres Endoskop (34, 34' 36, 36'), das in einem Endoskop-Betriebszustand zum Untersuchen von Fluoreszenzlicht in einem definierten weiteren Wellenlängenbereich eines zu Fluoreszenz angeregten weiteren Farbstoffs (NaFI) in dem Operationsbereich (22) und/oder das Untersuchen von Autofluoreszenzlicht in dem definierten weiteren Wellenlängenbereich von biologischem Gewebe in dem Operationsbereich (22) eingerichtet ist, wobei das Operationsmikroskop (12) in dem von dem ersten Betriebszustand verschiedenen weiteren Operationsmikroskop-Betriebszustand das Untersuchen von Fluoreszenzlicht in dem definierten weiteren Wellenlängenbereich des zu Fluoreszenz angeregten weiteren Farbstoffs (NaFI) in dem Operationsbereich (22) und/oder zum Untersuchen einer Autofluoreszenz in dem definierten weiteren Wellenlängenbereich von biologischem Gewebe und/oder Objekten in dem Operationsbereich (22) eingerichtet ist, und wobei das Operationsmikroskop (12) und das weitere Endoskop (34, 34' 36, 36') derart wirkungsgekoppelt sind, dass bei einem Betreiben des weiteren Endoskops (34, 34', 36, 36') in dem weiteren Endoskop-Betriebszustand der weitere Operationsmikroskop-Betriebszustand automatisch eingestellt wird.

8. Visualisierungssystem nach Anspruch 7, **gekennzeichnet durch** eine Einrichtung eingerichtet für das automatische Wirkungskoppeln des Operationsmikroskops (12) und des weiteren Endoskops (34, 34', 36, 36') bei einem Aktivieren des weiteren Endoskops (34, 34', 36, 36') und/oder bei einem Aufnehmen des weiteren Endoskops (34, 34', 36, 36') durch die Beobachtungsperson und/oder bei einem Anordnen eines Abschnitts des weiteren Endoskops (34, 34', 36, 36') in einem Beobachtungsbereich des Operationsmikroskops (12) und/oder bei einem Auftreten von Fluoreszenzlicht und/oder Autofluoreszenzlicht in dem definierten weiteren Wellenlängenbereich.

9. Visualisierungssystem nach einem der Ansprüche 1 bis 8, **gekennzeichnet durch** ein weiteres Endoskop (36, 36'), das in einem Endoskop-Betriebszustand zum Untersuchen von Fluoreszenzlicht in einem definierten weiteren Wellenlängenbereich eines zu Fluoreszenz angeregten weiteren Farbstoffs (ICG) in dem Operationsbereich (22) und/oder das Untersuchen von Autofluoreszenzlicht in dem definierten weiteren Wellenlängenbereich von biologischem Gewebe und/oder Objekten in dem Operationsbereich (22) eingerichtet ist, wobei das Operationsmikroskop (12) in einem von dem ersten Betriebszustand verschiedenen weiteren Operationsmikroskop-Betriebszustand zum Untersuchen von Fluoreszenzlicht in dem definierten weiteren Wellenlängenbereich des zu Fluoreszenz angeregten weiteren Farbstoffs (ICG) in dem Operationsbereich (22) und/oder das Untersuchen einer Autofluoreszenz in dem definierten Wellenlängenbereich von biologischem Gewebe und/oder Objekten in dem Operationsbereich (22) eingerichtet ist, und wobei das Operationsmikroskop (12) und das Endoskop (34, 34', 36, 36') derart wirkungsgekoppelt sind, dass bei einem Betreiben des Endoskops (34, 34', 36, 36') in diesem weiteren Endoskop-Betriebszustand der weitere Operationsmikroskop-Betriebszustand automatisch eingestellt wird.

10. Visualisierungssystem nach Anspruch 9, **gekennzeichnet durch** eine Einrichtung eingerichtet für das automatische Wirkungskoppeln des Operationsmikroskops (12) und des weiteren Endoskops (36, 36') bei einem Aktivieren des weiteren Endoskops (36, 36') und/oder bei einem Aufnehmen des weiteren Endoskops (36, 36') durch die Beobachtungsperson und/oder bei einem Anordnen eines Abschnitts des weiteren Endoskops (34, 34' 36, 36') in einem Beobachtungsbereich des Operationsmikroskops (12) und/oder bei einem Auftreten von Fluoreszenzlicht und/oder Autofluoreszenzlicht in dem definierten weiteren Wellenlängenbereich.

11. Visualisierungssystem nach einem der Ansprüche 1 bis 10, **dadurch gekennzeichnet, dass** das wenigstens eine Endoskop als ein Video-Endoskop (32, 32', 34, 34' 36, 36) ausgebildet ist.

12. Visualisierungssystem nach einem der Ansprüche 1 bis 11, **gekennzeichnet durch** eine Einrichtung (30) eingerichtet für das Drehen eines an dem Display (20) angezeigten endoskopischen Bilds aus dem Operationsbereich (22) relativ zu dem Display (20).

## Claims

1. Visualization system (10, 10')
with an operating microscope (12) configured for observing an operating region (22) with magnification, containing a computer unit (19) with a display (20) configured for displaying image data; and
with a device (30) configured for acquiring endoscopic images, which is operably coupled to the operating microscope (12) and which has at least one endoscope (32, 32', 32");
and with a circuit (66) which is designed for acquiring actuation information and actuatable by an observing person, said circuit setting an operating microscope operating state matched to the device (30) for acquiring endoscopic images in the operating region (22) when the actuation information for the operating microscope (12) is present;
**characterized in that**
the operating microscope (12), in a first operating microscope operating state, is configured for examining white light scattered in the operating region (22) and, in a further operating microscope operating state differing from the first operating state, is configured for examining fluorescence light in a defined wavelength range of a first dye (5-ALA) excited to fluoresce in the operating region (22) and/or for examining autofluorescence in the defined wavelength range of biological tissue and/or objects in the operating region (22), wherein the endoscope (32, 32', 34, 34', 36, 36'), in an endoscope operating state, is configured for examining fluorescence light in the defined wavelength range of the first dye (5-ALA) excited to fluoresce in the operating region (22) and/or for examining autofluorescence light in the defined wavelength range of biological tissue in the operating region (22), and wherein the operating microscope (12) and the endoscope (32, 32', 34, 34', 36, 36') are operably coupled in such a way that, when the endoscope (32, 32', 34, 34', 36, 36') is operated in this endoscope operating state, the further operating microscope operating state is set automatically.

2. Visualization system according to Claim 1, **characterized in that** the circuit is an activation circuit (66) which brings the device for acquiring the endoscopic images from a rest state into an activation state, wherein the activation circuit (66) has an activation sensor from the group containing gyro sensor, Hall sensor, touch sensor (68) or speech sensor.

3. Visualization system according to Claim 2 or 3, **characterized in that** the endoscope (32, 32', 32") has an endoscope body (39), in which the activation sensor (66) is arranged.

4. Visualization system according to Claim 2, **characterized in that** the activation sensor is arranged in the operating microscope (12).

5. Visualization system according to one of Claims 1 to 4, **characterized in that** the at least one endoscope (32, 32'), in a first endoscope operating state, is configured for examining white light scattered in the operating region (22) and, in a further endoscope operating state differing from the first endoscope operating state, is configured for examining fluorescence light in a defined wavelength range of a dye (5-ALA, NaFl, ICG) excited to fluoresce in the operating region (22) and/or for examining autofluorescence light in the defined wavelength range of biological tissue and/or objects in the operating region (22).

6. Visualization system according to one of Claims 1 to 5, **characterized by** a device configured for automatically operably coupling the operating microscope (12) with the at least one endoscope (32, 32') when the endoscope (32, 32', 34, 34', 36, 36') is activated and/or when the endoscope (32, 32', 34, 34', 36, 36') is picked up by the observing person and/or when a portion of the endoscope (32, 32', 34, 34', 36, 36') is arranged in an observation region of the operating microscope (12) and/or when fluorescence light and/or autofluorescence light in the defined wavelength range occurs.

7. Visualization system according to one of Claims 1 to 6, **characterized by** a further endoscope (34, 34', 36, 36') which, in an endoscope operating state, is configured for examining fluorescence light in a defined further wavelength range of a further dye (NaFl) excited to fluoresce in the operating region (22) and/or for examining autofluorescence light in the defined further wavelength range of biological tissue in the operating region (22), wherein the operating microscope (12), in the further operating microscope operating state differing from the first operating state, is configured for examining fluorescence light in the defined further wavelength range of the further dye (NaFl) excited to fluoresce in the operating region (22) and/or for examining autofluorescence in the defined further wavelength range of biological tissue and/or objects in the operating region (22), and wherein the operating microscope (12) and the further endoscope (34, 34', 36, 36') are operably coupled in such a way that, when the further endoscope (34, 34', 36, 36') is operated in the further endoscope operating state, the further operating microscope operating state is set automatically.

8. Visualization system according to Claim 7, **characterized by** a device configured for automatically operably coupling the operating microscope (12) and the further endoscope (34, 34', 36, 36') when the further endoscope (34, 34', 36, 36') is activated and/or when the further endoscope (34, 34', 36, 36') is picked up by the observing person and/or when a portion of the further endoscope (34, 34', 36, 36') is arranged in an observation region of the operating microscope (12) and/or when fluorescence light and/or autofluorescence light in the defined further wavelength range occurs.

9. Visualization system according to one of Claims 1 to 8, **characterized by** a further endoscope (36, 36') which, in an endoscope operating state, is configured for examining fluorescence light in a defined further wavelength range of a further dye (ICG) excited to fluoresce in the operating region (22) and/or for examining autofluorescence light in the defined further wavelength range of biological tissue and/or objects in the operating region (22), wherein the operating microscope (12), in a further operating microscope operating state differing from the first operating state, is configured for examining fluorescence light in the defined further wavelength range of the further dye (ICG) excited to fluoresce in the operating region (22) and/or for examining autofluorescence in the defined wavelength range of biological tissue and/or objects in the operating region (22), and wherein the operating microscope (12) and the endoscope (34, 34', 36, 36') are operably coupled in such a way that, when the endoscope (34, 34', 36, 36') is operated in this further endoscope operating state, the further operating microscope operating state is set automatically.

10. Visualization system according to Claim 9, **characterized by** a device configured for automatically operably coupling the operating microscope (12) and the further endoscope (36, 36') when the further endoscope (36, 36') is activated and/or when the further endoscope (36, 36') is picked up by the observing person and/or when a portion of the further endoscope (34, 34', 36, 36') is arranged in an observation region of the operating microscope (12) and/or when fluorescence light and/or autofluorescence light in the defined further wavelength range occurs.

11. Visualization system according to one of Claims 1 to 10, **characterized in that** the at least one endoscope is embodied as a video endoscope (32, 32', 34, 34', 36, 36').

12. Visualization system according to one of Claims 1 to 11, **characterized by** a device (30) configured for rotating an endoscopic image of the operating region (22) displayed at the display (20) relative to the display (20).

## Revendications

1. Système de visualisation (10, 10')
comportant un microscope chirurgical (12) conçu pour observer une zone opératoire (22) avec un certain grossissement, qui contient une unité de calcul (19) munie d'un dispositif d'affichage (20) conçu pour afficher des données d'image ; et
un dispositif (30) conçu pour détecter des images endoscopiques, qui est fonctionnellement couplé au microscope chirurgical (12) et qui comporte au moins un endoscope (32, 32', 32") ;
et comportant un circuit de commutation (66) conçu pour détecter une information d'actionnement et pouvant être actionné par un observateur qui, lors de la présence de l'information d'actionnement destinée au microscope chirurgical (12), règle un état de fonctionnement du microscope chirurgical sur le dispositif (30) adapté à l'acquisition d'images endoscopiques ;
**caractérisé en ce que**
le microscope chirurgical (12), dans un premier état de fonctionnement du microscope chirurgical, est conçu pour examiner une lumière blanche diffusée dans la zone opératoire (22), et qui, dans un autre état de fonctionnement du microscope chirurgical différent du premier état de fonctionnement, est conçu pour examiner la lumière de fluorescence dans une plage de longueurs d'onde définie d'un premier colorant (5-ALA) excité à fluorescence dans la zone opératoire (22) et/ou pour examiner une autofluorescence dans la plage de longueurs d'onde définie de tissus et/ou d'objets biologiques dans la zone opératoire (22), dans lequel l'endoscope (32, 32', 34, 34', 36, 36'), dans un état de fonctionnement de l'endoscope, est conçu pour examiner la lumière de fluorescence dans la plage de longueurs d'onde définie du premier colorant (5-ALA) excité à fluorescence dans la zone opératoire (22) et/ou pour examiner la lumière d'autofluorescence dans la plage de longueurs d'onde définie de tissus biologiques dans la zone opératoire (22), et dans lequel le microscope chirurgical (12) et l'endoscope (32, 32', 34, 34', 36, 36') sont fonctionnellement couplés de manière à ce que, lors d'un fonctionnement de l'endoscope (32, 32', 34, 34', 36, 36') dans ledit état de fonctionnement de l'endoscope, l'autre état de fonctionnement du microscope chirurgical soit automatiquement mis en œuvre.

2. Système de visualisation selon la revendication 1, **caractérisé en ce que** le circuit de commutation est un circuit d'activation (66) qui fait passer le dispositif destiné à détecter les images endoscopiques d'un état de repos à un état d'activation, dans lequel le circuit d'activation (66) comporte un capteur d'activation appartenant au groupe constitué d'un capteur gyroscopique, d'un capteur à effet Hall, d'un capteur tactile (68) ou d'un capteur vocal.

3. Système de visualisation selon la revendication 2 ou 3, **caractérisé en ce que** l'endoscope (32, 32', 32") comporte un corps d'endoscope (39) dans lequel est disposé le capteur d'activation (66).

4. Système de visualisation selon la revendication 2, **caractérisé en ce que** le capteur d'activation est disposé dans le microscope chirurgical (12).

5. Système de visualisation selon l'une quelconque des revendications 1 à 4, **caractérisé en ce que** ledit au moins un endoscope (32, 32'), dans un premier état de fonctionnement de l'endoscope, est conçu pour examiner la lumière blanche diffusée dans la zone opératoire (22) et, dans un autre état de fonctionnement de l'endoscope qui est différent du premier état de fonctionnement de l'endoscope, pour examiner la lumière de fluorescence dans une plage de longueurs d'onde définie d'un colorant (5-ALA, NaFl, ICG) excité à fluorescence dans la zone opératoire (22) et/ou pour examiner la lumière d'autofluorescence dans la plage de longueurs d'onde définie de tissus et/ou d'objets biologiques dans la zone opératoire (22).

6. Système de visualisation selon l'une quelconque des revendications 1 à 5, **caractérisé par** un dispositif conçu pour le couplage fonctionnel automatique du microscope chirurgical (12) avec ledit au moins un endoscope (32, 32') lors d'une activation de l'endoscope (32, 32', 34, 34', 36, 36') et/ou lors de la réception de l'endoscope (32, 32', 34, 34', 36, 36') par l'observateur et/ou lors d'une mise en place d'une partie de l'endoscope (32, 32', 34, 34', 36, 36') dans une zone d'observation du microscope chirurgical (12) et/ou lors d'une apparition de lumière de fluorescence et/ou de lumière d'autofluorescence dans la plage de longueurs d'onde définie.

7. Système de visualisation selon l'une des revendications 1 à 6, **caractérisé par** un autre endoscope (34, 34', 36, 36') qui, dans un état de fonctionnement de l'endoscope, est conçu pour examiner la lumière de fluorescence dans une autre plage de longueurs d'onde définie d'un autre colorant (NaFl) excité à fluorescence dans la zone opératoire (22) et/ou pour examiner la lumière d'autofluorescence dans l'autre plage de longueurs d'onde définie de tissus biologiques dans la zone opératoire (22), dans lequel le microscope chirurgical (12), dans l'autre état de fonctionnement du microscope chirurgical qui est différent du premier état de fonctionnement, est conçu pour examiner la lumière de fluorescence dans l'autre plage de longueurs d'onde définie de l'autre colorant (NaFl) excité à fluorescence dans la zone opératoire (22) et/ou pour examiner une autofluorescence dans l'autre plage de longueurs d'onde définie de tissus et/ou d'objets biologiques dans la zone opératoire (22), et dans lequel le microscope chirurgical (12) et l'autre endoscope (34, 34', 36, 36') sont fonctionnellement couplés de manière à ce que, lors d'un fonctionnement de l'autre endoscope (34, 34', 36, 36') dans l'autre état de fonctionnement de l'endoscope, l'autre état de fonctionnement du microscope chirurgical soit automatiquement mis en œuvre.

8. Système de visualisation selon la revendication 7, **caractérisé par** un dispositif conçu pour le couplage fonctionnel automatique du microscope chirurgical (12) et de l'autre endoscope (34, 34', 36, 36') lors d'une activation de l'autre endoscope (34, 34', 36, 36') et/ou lors de la réception de l'autre endoscope (34, 34', 36, 36') par l'observateur et/ou lors d'une mise en place d'une partie de l'autre endoscope (34, 34', 36, 36') dans une zone d'observation du microscope chirurgical (12) et/ou lors d'une apparition de lumière de fluorescence et/ou de lumière d'autofluorescence dans l'autre plage de longueurs d'onde définie.

9. Système de visualisation selon l'une des revendications 1 à 8, **caractérisé par** un autre endoscope (36, 36') qui, dans un état de fonctionnement de l'endoscope, est conçu pour examiner la lumière de fluorescence dans une autre plage de longueurs d'onde définie d'un autre colorant (ICG) excité à fluorescence dans la zone chirurgicale (22) et/ou pour examiner la lumière d'autofluorescence dans l'autre plage de longueurs d'onde définie de tissus et/ou d'objets biologiques dans la zone opératoire (22), dans lequel le microscope chirurgical (12), dans un autre état de fonctionnement du microscope chirurgical qui est différent du premier état de fonctionnement, est conçu pour examiner la lumière de fluorescence dans l'autre plage de longueurs d'onde définie de l'autre colorant (ICG) excité à fluorescence dans la zone opératoire (22) et/ou pour examiner une autofluorescence dans la plage de longueurs d'onde définie de tissus et/ou d'objets biologiques dans la zone opératoire (22), et dans lequel le microscope chirurgical (12) et l'endoscope (34, 34', 36, 36') sont fonctionnellement couplés de manière à ce que, lors d'un fonctionnement de l'endoscope (34, 34', 36, 36') dans ledit autre état de fonctionnement de l'endoscope, l'autre état de fonctionnement du microscope chirurgical soit automatiquement mis en œuvre.

10. Système de visualisation selon la revendication 9, **caractérisé par** un dispositif conçu pour le couplage fonctionnel automatique du microscope chirurgical (12) et de l'autre endoscope (36, 36') lors d'une activation de l'autre endoscope (36, 36') et/ou lors de la réception de l'autre endoscope (36, 36') par l'observateur et/ou lors d'une mise en place d'une partie de l'autre endoscope (34, 34', 36, 36') dans une zone d'observation du microscope chirurgical (12) et/ou lors d'une apparition de lumière de fluorescence et/ou de lumière d'autofluorescence dans l'autre plage de longueurs d'onde définie.

11. Système de visualisation selon l'une des revendications 1 à 10, **caractérisé en ce que** ledit au moins un endoscope est réalisé sous la forme d'un endoscope vidéo (32, 32', 34, 34', 36, 36').

12. Système de visualisation selon l'une des revendications 1 à 11, **caractérisé par** un dispositif (30) conçu pour faire tourner par rapport au système d'affichage (20) une image endoscopique provenant de la zone opératoire (22) et affichée sur le dispositif d'affichage (20).
